# EUROPÄISCHE PATENTANMELDUNG

(11) **EP 2 140 909 A1**
(43) Veröffentlichungstag der Anmeldung: **06.01.2010**
(21) Anmeldenummer: 08159538.1
(22) Anmeldetag: 02.07.2008
(51) Int. Cl.: A61N 1/375

(54) **Medizinisches Implantat**

(71) Anmelder: Sorin Group Deutschland GmbH, 80939 München (DE)
(72) Erfinder: Hahn, Andreas, 82335 Berg (DE)
(74) Vertreter: HOFFMANN EITLE

(57) **Zusammenfassung**

Ein medizinisches Implantat, insbesondere Herzschrittmacher, Kardioverter, Defibrillator oder dergleichen, mit zumindest einer funktionellen Einheit (2, 4), wie beispielsweise ein Energiespeicher (2), eine Steuerelektronik (4) oder Kombinationen daraus, und einem Gehäuse (20) zur Aufnahme der zumindest einen funktionellen Einheit (2, 4). Das medizinische Implantat umfasst ferner zumindest eine Elektrode (10), die ein Anschlussende (12), eine Elektrodenleitung (11) und ein Stimulierungsende (13) aufweist, wobei das Anschlussende (12) der Elektrode (10) in dem Gehäuse (20) zur Aufnahme der zumindest einen funktionellen Einheit (2, 4) angeordnet ist.

## Beschreibung

### Technisches Gebiet

Die Erfindung betrifft ein medizinisches Implantat, insbesondere Herzschrittmacher, Kardioverter, Defibrillator oder dergleichen, nach dem Oberbegriff von Anspruch 1.

### Stand der Technik

Bekannte medizinische Implantate, wie beispielsweise Herzschrittmacher, Kardioverter oder Defibrillatoren, umfassen üblicherweise ein fluiddichtes, d.h. flüssigkeits- und gasdichtes Gehäuse. In dem Gehäuse sind zumindest eine Steuerelektronik und ein langlebiger Energiespeicher zur Versorgung der Elektronik untergebracht. Ferner ist an dem Gehäuse ein Anschlussgehäuse (Header) angeordnet, das genormte Anschlussbuchsen zum Anschließen von Elektroden aufweist. Die Anschlussbuchsen weisen Kontakte zur elektrischen Verbindung der Elektroden mit der Steuerelektronik im Innern des Gehäuses auf.

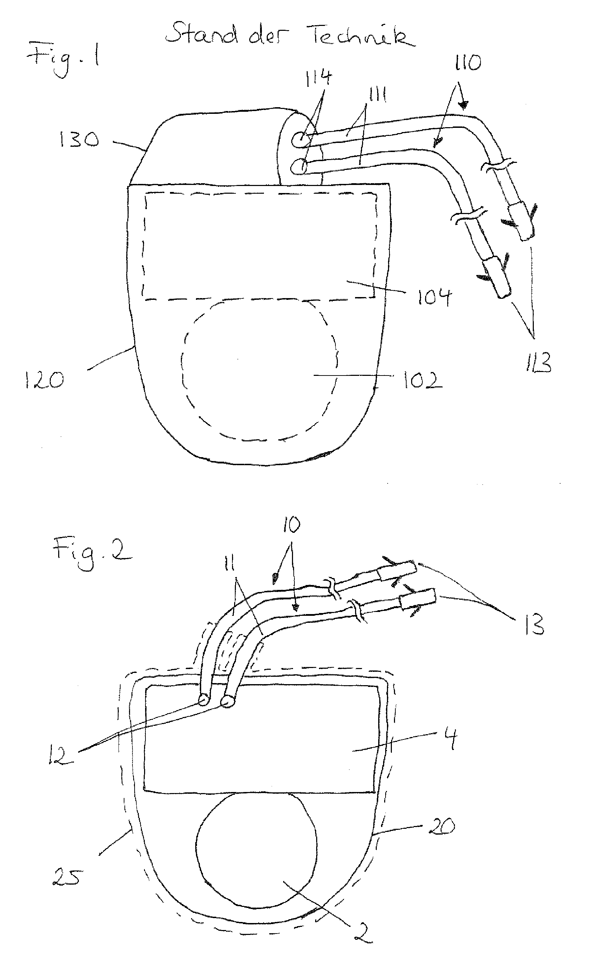

Ein derartiges medizinisches Implantat wird schematisch in Fig. 1 gezeigt. Wie in Fig. 1 zu sehen ist, weist das medizinische Implantat ein Gehäuse 120 auf, in dem eine Steuerelektronik 104 und ein Energiespeicher 102 angeordnet sind. Ferner ist an der Oberseite des Gehäuses 120 ein Anschlussgehäuse 130 mit Anschlussbuchsen 114 zur elektrischen Verbindung von Elektroden 110 mit der Steuerelektronik 104 im Innern des Gehäuses angeordnet. Zum Herstellen einer solchen elektrischen Verbindung wird jeweils ein entsprechendes Anschlussende (nicht gezeigt) einer Elektrodenleitung 111 in die Anschlussbuchse 114 gesteckt. An dem entgegengesetzten Ende der Elektrodenleitung 111 zu dem Anschlussende ist jeweils ein Stimulierungsende 113 vorgesehen, das im Herzmuskel eines Patienten zu platzieren ist. Das Gehäuse 120 und das Anschlussgehäuse 130 ergeben zusammen eine fluiddichte Gehäuseanordnung, wobei die Steuerelektronik 104 im Innern des Gehäuses 120 über Zuleitungen mit Kontakten (nicht gezeigt) der Anschlussbuchsen 114 verbunden ist. Für die Montage des Anschlussgehäuses 130 am Gehäuse 120 werden zur fluiddichten elektrischen Verbindung im Allgemeinen spezielle Durchführungen (nicht gezeigt) verwendet.

An bekannte medizinische Implantate werden hohe Qualitätsanforderungen gestellt. Insbesondere müssen das Gehäuse und das Anschlussgehäuse im Allgemeinen über die Dauer von rund zehn Jahren zusammen eine fluiddichte Gehäuseanordnung bilden, um die Funktionalität des Implantats sicherzustellen und eine Gesundheitsgefährdung des Patienten auszuschließen. Hierzu ist auch eine über die Jahre fluiddicht bleibende Steckverbindung der Elektroden am Anschlussgehäuse erforderlich. Ferner muss diese Steckverbindung auch einer dauerhaft dynamischen Belastung durch die Bewegungen des Patienten standhalten. Um unter den gegebenen Umständen über die gesamte Lebensdauer fluiddicht zu sein, weisen das Anschlussgehäuse und die zur fluiddichten elektrischen Verbindung verwendeten Durchführungen einen entsprechend aufwendigen Aufbau auf, was zu hohen Herstellungskosten des medizinischen Implantats beiträgt. Aufgrund des aufwendigen Aufbaus nimmt Anschlussgehäuse einen relativ großen Anteil, im Allgemeinen ca. 1/3, der Gesamtabmessung des medizinischen Implantats ein.

### Darstellung der Erfindung

Es ist daher Aufgabe der vorliegenden Erfindung ein medizinisches Implantat bereitzustellen, das eine verkleinerte Gesamtabmessung aufweist und preiswert herzustellen ist.

Diese Aufgabe wird erfindungsgemäß durch ein medizinisches Implantat nach Anspruch 1 gelöst.

Gemäß einem Aspekt der vorliegenden Erfindung wird ein medizinisches Implantat bereitgestellt, insbesondere Herzschrittmacher, Kardioverter, Defibrillator oder dergleichen, mit zumindest einer funktionellen Einheit, wie beispielsweise ein Energiespeicher, eine Steuerelektronik oder Kombinationen daraus; einem Gehäuse zur Aufnahme der zumindest einen funktionellen Einheit; und zumindest einer Elektrode, die ein Anschlussende, eine Elektrodenleitung und ein Stimulierungsende aufweist. Bei dem medizinischen Implantat ist das Anschlussende der Elektrode in dem Gehäuse zur Aufnahme der zumindest einen funktionellen Einheit angeordnet.

Durch die erfindungsgemäße Anordnung des Anschlussendes der Elektrode direkt in dem Gehäuse zur Aufnahme der zumindest einen funktionellen Einheit kann auf ein separates Anschlussgehäuse verzichtet werden. Hierdurch verringert sich die Gesamtabmessung des medizinischen Implantats um ca. 1/3 im Vergleich mit entsprechenden Implantaten des Standes der Technik. Ferner sind durch die erfindungsgemäße Ausgestaltung keine separaten Durchführungen zur Herstellung einer fluiddichten elektrischen Verbindung zwischen Gehäuse und Anschlussgehäuse notwendig. Da kein aufwendig herzustellendes Anschlussgehäuse und auch keine elektrischen Durchführungen erforderlich sind, verringern sich außerdem die Herstellungskosten des medizinischen Implantats.

Vorzugsweise ist das medizinische Implantat in Form eines Kardio-Implantats ausgeführt, wie beispielsweise ein Herzschrittmacher, Kardioverter oder Defibrillator.

In dem Gehäuse des medizinischen Implantats ist zumindest eine funktionelle Einheit aufgenommen, wie beispielsweise ein Energiespeicher, eine Steuerelektronik oder Kombinationen daraus. Vorzugsweise sind in dem Gehäuse zumindest zwei funktionelle Einheiten in Form eines Energiespeichers und einer Steuerelektronik aufgenommen. Der in dem Gehäuse aufgenommene Energiespeicher liefert hierbei einen Gleichstrom, der von der Steuerelektronik in medizinisch nutzbare Impulse oder Felder umgewandelt wird, die dann über die Elektroden an den Herzmuskel des Patienten übertragen werden.

Das medizinische Implantat umfasst zumindest eine, vorzugsweise zwei bis vier Elektroden, deren jeweiliges Anschlussende direkt in dem Gehäuse zur Aufnahme der zumindest einen funktionellen Einheit angeordnet ist. Vorzugsweise ist das Anschlussende der Elektrode direkt an der entsprechenden funktionellen Einheit, z.B. der Steuerelektronik, angebracht. Die direkte Verbindung des Anschlussendes der Elektrode mit der Steuerelektronik erfolgt vorzugsweise über Bonden oder eine Lötverbindung, was beides eine preiswerte und einfach auszuführende Art der Verbindung darstellt. Ferner wird jedoch auch in Erwägung gezogen, das Anschlussende der Elektrode indirekt, z.B. durch eine Steckverbindung, mit der Steuerelektronik zu verbinden, so dass beispielsweise der Schritt des Lötens entfällt.

Vorzugsweise wird das medizinische Implantat mittels Telemetrie ausgelesen und über externe Geräte programmiert. Telemetrie, oder auch Fernmessung, bezeichnet hierbei die Übertragung von Messwerten eines am Messort befindlichen Messfühlers (Sensor) zu einer räumlich getrennten Stelle. Bei einer Programmierung über externe Geräte werden Daten von einer ersten Einrichtung an eine räumlich getrennte zweite Einrichtung übertragen, wobei die Daten Programmanweisungen zur Ausführung durch die zweite Einrichtung umfassen. Beispielsweise erfolgt eine Neuprogrammierung bzw. Abfrage von Daten des medizinischen Implantats mit Hilfe eines Progammiergerätes mit einem Programmierkopf, der auf die Haut des Patienten über der Position des medizinischen Implantats gelegt wird. Der Programmierkopf umfasst eine Station mit Ladefunktion zum Auslesen und Programmieren von Daten. Dabei werden Parameter sowie diagnostische Werte des medizinischen Implantats übertragen und ausgewertet. Anschließend werden die aktualisierten Parameter in das medizinische Implantat zurückgeladen. Für eine Funktionskontrolle bzw. Neuprogrammierung des medizinischen Implantats ist somit kein erneuter operativer Eingriff am Patienten notwendig.

Gemäß einer Weiterbildung der Erfindung ist das Gehäuse einstückig ausgebildet. In diesem Fall wird das Gehäuse direkt um die zumindest eine funktionelle Einheit und das Anschlussende der zumindest einen Elektrode herum ausgebildet, wobei es vorzugsweise durch Urformen um die funktionelle Einheit und das Anschlussende herum ausgebildet wird, insbesondere durch Gießen, wie z.B. Spritzgießen. Hierzu können die zumindest eine funktionelle Einheit und das entsprechende Anschlussende der zumindest einen Elektrode in einer Gussform umgossen bzw. umspritzt werden. Zur relativen Fixierung der einzelnen Komponenten zueinander während des Gießvorgangs, kann die Gussform entsprechende Aussparungen, Stege o.ä. aufweisen. Das Stimulierungsende der Elektrode und ein wesentlicher Teil der Elektrodenleitung werden während des Gießvorgangs außerhalb der Gussform angeordnet. Die einteilige Ausbildung des Gehäuses hat den Vorteil, dass keine weiteren Fügevorgänge zur Verbindung mehrerer Gehäuseteile erforderlich sind. Dies trägt weiter zur Reduzierung der Kosten des medizinischen Implantats bei.

Bei einer anderen vorteilhaften Ausführungsform der vorliegenden Erfindung umfasst das Gehäuse zumindest zwei Gehäuseteile. Die zumindest zwei Gehäuseteile werden beispielsweise mittels Gießen, vorzugsweise mittels Spritzgießen, gefertigt und anschließend werden die benötigten funktionellen Einheiten in die "vorgefertigten" Gehäuseteile eingelegt. Das jeweilige Anschlussende der benötigten Elektroden kann vor dem Einlegen in die Gussform oder danach an die entsprechende funktionelle Einheit gefügt werden, beispielsweise mittels einer Lötverbindung. Das Gehäuse kann jedoch auch mehr als zwei Gehäuseteile umfassen, je nach Fertigungs- und/oder Anwendungserfordernissen. Vorzugsweise umfasst zumindest ein Gehäuseteil des vorgefertigten Gehäuses eine Öffnung zur Durchführung der Elektrodenleitung(en). Da die einzelnen Gehäuseteile vor dem Einlegen der benötigten Komponenten separat gefertigt werden können, vereinfacht sich die Ausgestaltung der Gussform, da beispielsweise kein Auslass zum Herausführen von Elektrodenleitungen vorgesehen werden muss.

Vorzugsweise weisen die Gehäuseteile an der Gehäuseinnenseite zumindest eine Struktur zur Montage der zumindest einen funktionellen Einheit auf. Die Struktur zur Montage der zumindest einen funktionellen Einheit kann beispielsweise Aussparungen, Stege, Rahmen usw. umfassen, die das Positionieren beim Einsetzen sowie das Fixieren der verschiedenen Komponenten am Platz im Gehäuse erleichtern. Ferner können auch Zentrierelemente, z.B. Vertiefungen oder Vorsprünge, vorgesehen werden, die ein passgenaues Zusammenfügen der einzelnen Gehäuseteile sicherstellen.

Bei einer mehrteiligen Ausgestaltung des Gehäuses kann dieses beispielsweise aus zwei Edelstahl- bzw. Titan-Halbschalen ausgebildet werden, die fluiddicht verbunden werden. Zur fluiddichten Verbindung können die Titan-Halbschalen beispielsweise zunächst mittels eines Laser-Heftprozesses in genauer Lage zueinander fixiert und anschließend die umlaufende Naht verschweißt werden.

Vorzugsweise ist das Gehäuse jedoch aus Kunststoff ausgebildet. Kunststoff eignet sich sowohl zur Fertigung eines einstückigen Gehäuses als auch eines mehrteiligen Gehäuses. Vorzugsweise ist der Kunststoff ein biokompatibler Kunststoff, wie zum Beispiel Polyurethan. Gehäuse bzw. Gehäuseteile aus Kunststoffmaterialien können sehr preiswert, z.B. durch Spritzgussverfahren, hergestellt werden. Im Fall eines mehrteiligen Kunststoffgehäuses können die einzelnen Gehäuseteile einfach und preiswert miteinander verbunden werden, z.B. durch Kleben, vorzugsweise mit Epoxid, Ultraschall-Schweißen oder thermisches Fügen.

Um den mechanischen und elektrisch isolierenden Anforderungen bei medizinischen Implantaten gerecht zu werden, sollte eine bestimmte Wandstärke des Kunststoffgehäuses nicht unterschritten werden. Bei Verwendung von Polyurethan beträgt die Wandstärke vorzugsweise mindestens 0,5 mm.

Das Gehäuse kann jedoch auch andere biokompatible Materialien umfassen. Beispielsweise kann das Gehäuse eine Maschenware umfassen, auf die im Folgenden mit "Fliegengitter" Bezug genommen wird. Das Fliegengitter weist vorzugsweise eine Maschenweite von ca. 1 mm auf. Die Maschenweite ist auf die zu schirmende Wellenlänge abzustimmen und sollte etwa 1/10tel dieser Wellenlänge betragen. Übliche zum Einsatz kommende Maschenweiten werden zwischen 0,5 mm und 5 mm liegen. Vorteilhafterweise ist das Fliegengitter aus Metall hergestellt. Bei Verwendung eines derartigen Fliegengitters als das Gehäuse, kann dieses einteilig oder mehrteilig ausgeführt werden. Bei einer mehrteiligen Ausführung werden die einzelnen Gehäuseteile vorzugsweise aneinander gefügt, zum Beispiel durch zumindest teilweises Verhaken bzw. Verklemmen oder Verschweißen/Verlöten der jeweiligen Randgebiete der einzelnen Gehäuseteile. Die Verwendung eines Fliegengitters stellt eine preiswerte Alternative beispielsweise zu spritzgegossenen Kunststoff-Gehäusen dar.

Gemäß einer vorteilhaften Weiterbildung der Erfindung ist das Gehäuse zumindest teilweise mit einem elektrisch leitfähigen Material versehen. Das elektrisch leitfähige Material wird an dem Gehäuse angeordnet, damit es eine abschirmende Wirkung gegen elektrische Störfelder hat, so dass die elektromagnetische Verträglichkeit (EMV) gewährleistet ist. Im Fall von vorgefertigten Gehäuseteilen, wird das elektrisch leitfähige Material vorzugsweise an der Innenseite der jeweiligen Gehäuseteile vorgesehen, wohingegen im Fall eines einstückigen Gehäuses das elektrisch leitfähige Material vorzugsweise an der Außenseite des Gehäuses vorgesehen wird. Falls ein Fliegengitter aus einem elektrisch leitfähigen Metall als das Gehäuse verwendet wird, ist keine zusätzliche Anordnung eines elektrisch leitfähigen Materials nötig. Das Versehen des Gehäuses mit einem elektrisch leitfähigen Material kann beispielsweise durch Bedampfung mit Metall erfolgen oder durch Beschichtung mit leitfähigen Kunststoffen, wie beispielsweise Polythiophen.

Bei einer Weiterbildung des erfindungsgemäßen medizinischen Implantats sind das Stimulierungsende der Elektrode und zumindest ein Teil der Elektrodenleitung außerhalb des Gehäuses angeordnet, und das Gehäuse und zumindest ein Abschnitt des außerhalb des Gehäuses angeordneten Teils der Elektrodenleitung weisen eine Ummantelung auf. Die Ummantelung stellt sicher, dass das Gehäuse, auch im Bereich der Herausführung der Elektrodenleitung, eine fluiddichte Einheit bildet. Im Fall eines mehrteiligen Gehäuses oder der Verwendung eines Fliegengitters als das Gehäuse wird bevorzugt, das gesamte Gehäuse zu ummanteln. Es wird jedoch auch in Erwägung gezogen, nicht das gesamte Gehäuse zu ummanteln, sondern z.B. nur den Teil des Gehäuses, der an die Elektrodenleitung angrenzt. Eine Teilummantelung des Gehäuses bietet sich beispielsweise an, wenn das Gehäuse einstückig ausgeführt ist. Vorzugsweise umfasst das Gehäuse des medizinischen Implantats, insbesondere im Fall lediglich einer Teilummantelung, ein biokompatibles Material, wie z.B. Titan, Polyurethan oder Silikonkautschuk.

Vorzugsweise umfasst die Ummantelung ein biokompatibles Material, wie beispielsweise Silikonkautschuk. Silikon-Werkstoffe weisen im Allgemeinen eine höhere Biokompatibilität als beispielsweise Edelstahl auf, und erlauben im Vergleich zu einer Verwendung von Metallen eine weitere Reduzierung der Kosten und der Größe des medizinischen Implantats. Ferner gestatten Silikon-Werkstoffe eine Umspritzung des Gehäuses, die fertigungstechnisch einfach auszuführen und preiswert ist. Die Schichtdicke der Ummantelung wird in der Regel mindestens 0,5 mm betragen.

Gemäß einer bevorzugten Weiterbildung der vorliegenden Erfindung umfasst der Energiespeicher einen Akkumulator und/oder einen Kondensator. Der Energiespeicher kann hierbei entweder einen Akkumulator oder einen Kondensator oder eine Kombination aus Akkumulator und Kondensator umfassen. Vorzugsweise umfasst der Energiespeicher jedoch einen Akkumulator oder eine Akkumulator-Kondensator-Kombination.

Herkömmlicherweise werden für die Energieversorgung bei medizinischen Implantaten Lithium-Batterien eingesetzt, die eine Lebensdauer von ca. 10 Jahren aufweisen. Die Ladungsmenge, die eine Batterie speichern kann, wird als "Kapazität" (Nennkapaziät) bezeichnet und hat einen entsprechenden Einfluss auf die Größe der Batterie. Um diese lange Lebensdauer aufzuweisen, ist die Kapazität der Batterie entsprechend groß, was wiederum eine nicht zu vernachlässigende Abmessung der Batterie zur Folge hat. Daher nimmt auch die Batterie bei herkömmlichen medizinischen Implantaten ca. 1/3 der Gesamtabmessung des medizinischen Implantats ein.

Erfindungsgemäß wird jedoch anstelle einer Batterie ein wiederaufladbarer Akkumulator verwendet. Vorzugsweise weist der Akkumulator eine deutlich kleinere Gangreserve als die herkömmlicherweise verwendeten Batterien auf, und kann daher auch mit deutlich geringeren Außenabmessungen hergestellt werden. Hierbei weist der Akkumulator vorzugsweise eine Gangreserve kleiner ca. vier Jahre auf, bevorzugter kleiner ca. zwei Jahre, insbesondere bevorzugt eine Gangreserve von ca. einem Jahr. Analog zur Batterie, wird die Ladungsmenge, die ein Akkumulator speichern kann, in Ampèrestunden (Ah) angegeben und als Kapazität bezeichnet. Bei einem Akkumulator mit ca. einem Jahr Gangreserve beträgt die Kapazität ca. 1 Ah. Vorzugsweise beträgt die Kapazität des Akkumulators weniger als 300 mAh, bevorzugter weniger als 200 mAh, am meisten bevorzugt 100 mAh oder weniger. Für einen Ladevorgang mit 3 V und 100 mAh würden für das Laden des Akkumulators ungefähr 300 mW eingesetzt, so dass eine Gewebeschädigung beim Patienten vermieden werden kann. Durch die Reduzierung der Kapazität auf ungefähr ein Jahr Gangreserve, könnte der Akkumulator somit deutlich kleiner ausgeführt werden, was wiederum zu einer Verkleinerung der Gesamtabmaße des medizinischen Implantats beiträgt. Nebenbei angemerkt, hängt die Gangreserve bzw. der Aufladezeitraum des Akkumulators davon ab, ob die Steuerelektronik häufig Impulse abgeben muss oder nur selten, so dass die Größenreduzierung je nach Einsatzgebiet des medizinischen Implantats erheblich sein kann.

Wenn der Energiespeicher in Form eines Akkumulators ausgeführt ist, kann zusätzlich noch ein Kondensator vorgesehen werden. In diesem Fall dient der Kondensator als eine Art "Notfall-Energiespeicher", der einen Ausfall oder eine Fehlfunktion des medizinischen Implantats verhindert, wenn der Akkumulator nur noch eine sehr geringe Ladung aufweist bzw. entladen ist. Zeitgleich kann eine Warnfunktion ausgelöst werden, die dem Träger des medizinischen Implantats die Notwendigkeit der baldigen Aufladung anzeigt.

Vorzugsweise ist der Energiespeicher ausgestaltet, um mittels hochfrequenter Energie, magnetisch-induktiven Ladens, Wärme eines Patienten, chemischer Energie, mechanischer Bewegung oder Lungenbewegung wieder aufgeladen zu werden. Bezüglich einer Ladung mittels hochfrequenter Energie, könnte der Akkumulator eine Ladespule mit Ferritkern aufweisen, über die bei einer Frequenz von oberhalb der des Schrittmachers bis ca. 100 kHz kontaktlos geladen werden kann. Eine Aufladung mittels mechanischer Bewegung des Patienten erfolgt durch die Bewegung des Rumpfes des Patienten, beispielsweise beim Gehen, Laufen oder sich Niederbeugen. Eine Aufladung mittels Lungenbewegung (Atmen) hingegen erfolgt lediglich durch die inhärente Bewegung der Lunge, ohne dass der Patient hierzu seinen Körper insgesamt bewegen muss. Weiterhin könnte der Energiespeicher ausgestaltet sein, um mittels Kardio-Kontraktionen, Vaskulatur-Kontraktionen (pulsierender Blutstrom) oder Fluid-Strom geladen zu werden. Auch eine Umwandlung von Wärme eines Patienten in Strom, oder die Umwandlung von chemischer Energie von Zellen nahe der Implantationsstelle in elektrische Energie, wird in Erwägung gezogen. Die Möglichkeiten zur Ausgestaltung eines Akkumulator, um mittels hochfrequenter Energie, magnetisch-induktiven Ladens, Wärme eines Patienten, chemischer Energie, mechanischer Bewegung oder Lungenbewegung wieder aufgeladen zu werden, sind im Stand der Technik bekannt, so dass an dieser Stelle von einer näheren Erläuterung abgesehen wird.

Vorzugsweise umfasst die Steuerelektronik einen anwendungsspezifischen integrierten Schaltkreis (ASIC). Durch die Ausgestaltung als integrierter Schaltkreis ist die Funktion der Steuerelektronik nicht manipulierbar, weshalb die Herstellungskosten für einen ASIC gering sind. Ferner arbeiten ASICs aufgrund der Anpassung ihrer Architektur an ein spezifisches Problem sehr effizient, wodurch eine längere Lebensdauer des Energiespeichers gewährleistet wird. Überdies sind sie ohne Weiteres verfügbar, was vergleichsweise niedrige Kosten mit sich bringt. Durch das Vorsehen eines anwendungsspezifischen integrierten Schaltkreises können somit sowohl die Gesamtkosten des medizinischen Implantats reduziert als auch die Lebensdauer des Energiespeichers verlängert werden.

Gemäß einem anderen Aspekt der vorliegenden Erfindung wird ein Teilesatz bereitgestellt, der zumindest zwei medizinische Implantate, wie oben angegeben, umfasst. Die zumindest zwei medizinischen Implantate umfassen unterschiedliche Ausgestaltungen von Elektroden, insbesondere des jeweiligen Stimulierungsendes. Erfindungsgemäß werden hierbei komplette medizinische Implantate vorgesehen, die sich in der Ausgestaltung der Elektroden, insbesondere der jeweiligen Stimulierungsenden der Elektroden, unterscheiden. Beim bisher bekannten Stand der Technik wurden jeweils einheitliche medizinische Implantate vorgesehen, bei denen je nach Anwendungszweck unterschiedliche Elektroden in das separate Anschlussgehäuse eingeführt wurden. Aufgrund der erheblich reduzierten Herstellungskosten des erfindungsgemäßen medizinischen Implantats, beispielsweise durch Weglassen eines separaten Anschlussgehäuses und spezieller elektrischer Durchführungen, ist jedoch auch die Bevorratung mit kompletten bzw. sofort einsatzbereiten medizinischen Implantaten einschließlich der jeweiligen Elektroden wirtschaftlich akzeptabel. Durch das Bereitstellen eines Teilesatzes kann dem behandelnden Mediziner eine Auswahl an unmittelbar einsatzbereiten Implantaten für alle der üblichen Indikationen bereitgestellt werden.

Grundsätzlich gibt es zwei Arten von Elektroden, unipolare und bipolare Elektroden. Bei unipolaren Elektroden wird das entsprechende Signal nur in eine Richtung geleitet, während es bei bipolaren Elektroden zweipolig geleitet wird. Die Verankerung des Stimulierungsendes der Elektrode im Herzmuskel kann wiederum aktiv durchgeführt werden, z.B. durch Fixierung mittels Schraubenelektroden im Muskelgewebe, oder passiv, z.B. durch Festhaken mittels Ankerelektroden an den Muskelfasern. Ein Teilesatz umfassend zumindest zwei medizinische Implantate mit unterschiedlichen Ausgestaltungen von Elektroden, insbesondere des jeweiligen Stimulierungsendes, ist deshalb besonders vorteilhaft, da gleichzeitig einsatzbereite medizinische Implantate mit verschiedenen Elektroden für unterschiedliche Anwendungszwecke, je nach Positionierung der Elektrode im Herzen und Erkrankung des Patienten, bevorratet werden können.

### Kurze Beschreibung der Zeichnungen

Fig. 1 zeigt schematisch eine Vorderansicht eines medizinischen Implantats des Stands der Technik.
Fig. 2 zeigt schematisch eine Vorderansicht eines medizinischen Implantats gemäß der vorliegenden Erfindung.

### Ausführliche Beschreibung bevorzugter Ausführungsformen

Bevorzugte Ausführungsformen der vorliegenden Erfindung werden nachfolgend detailliert unter Bezugnahme auf die begleitende Zeichnung beschrieben.

In Fig. 2 wird schematisch eine Vorderansicht eines medizinischen Implantats gemäß der vorliegenden Erfindung gezeigt. Das medizinische Implantat weist ein Gehäuse 20 auf, in dem eine Steuerelektronik 4 und ein Energiespeicher 2 angeordnet sind. Der Energiespeicher 2 umfasst vorzugsweise einen wiederaufladbaren Akkumulator mit einer Kapazität ausreichend für ungefähr ein Jahr Gangreserve, wodurch der Akkumulator deutlich kleiner als beispielsweise der Energiespeicher 102 des medizinischen Implantats des Stands der Technik ausgeführt werden kann. Diese kleine Größe des Akkumulators 2 trägt wiederum zu einer Verkleinerung der Gesamtabmaße des medizinischen Implantats bei. Die Form des Akkumulators 2 ist jedoch nicht auf die in Fig. 2 gezeigte runde Form beschränkt, sondern er kann in jeder geeigneten Form ausgeführt sein, wie beispielsweise auch oval oder rechteckig.

Ferner umfasst die gezeigte Ausführungsform des erfindungsgemäßen medizinischen Implantats zwei Elektroden 10 mit jeweils einem Anschlussende 12, einer Elektrodenleitung 11 und einem Stimulierungsende 13, das an dem Herzmuskel eines zu behandelnden Patienten anzuordnen ist. Bei der in Fig. 2 gezeigten Ausführungsform, sind die Elektroden 10 als so genannte "Anker-Elektroden" dargestellt, wobei das jeweilige Stimulierungsende 13 am Herzmuskel des Patienten festgehakt wird. Die Art der Ausgestaltung der Elektroden 10 ist jedoch nicht auf diese Form beschränkt, sondern die Elektroden 10 und ihre entsprechenden Stimulierungsenden 13 können jede geeignete Form aufweisen. Das Anschlussende 12 der Elektrode 10 ist jeweils direkt mit der Steuerelektronik 4 verbunden. Die Verbindung des Anschlussendes 12 der Elektrode 10 mit der Steuerelektronik 4 kann hierbei beispielsweise mittels Bonden oder einer Lötverbindung durchgeführt werden. Durch die direkte Verbindung des Anschlussendes 12 der Elektrode 10 mit der Steuerelektronik 4 kann auf ein zusätzliches Anschlussgehäuse, wie es bei medizinischen Implantaten des Stands der Technik erforderlich ist, verzichtet werden. Da ein derartiges Anschlussgehäuse ca. 1/3 der Gesamtabmessung eines medizinischen Implantats einnimmt, führt das Weglassen dieses Anschlussgehäuses zu einer deutlichen Verringerung der Gesamtabmessung des medizinischen Implantats.

Wie man aus Fig. 2 sehen kann, sind das gesamte Gehäuse 20 sowie ein Teil der Elektrodenleitungsisolierungen 11 mit einer Ummantelung 25 versehen. Vorzugsweise sind hierbei die Elektrodenleitungsisolierungen 11 jeweils in einer Länge von ungefähr 1 cm bis 10 cm ummantelt. Diese Länge ist ausreichend, um auch bei dauerhaft dynamischer Belastung einen fluiddichten Abschluss zwischen Gehäuse 20 und Elektrodenleitungen 11 vorzusehen. Die jeweiligen Elektrodenleitungen 11 können hierbei einzeln (wie in Fig. 2 gezeigt), oder als eine Einheit insgesamt mit dem Gehäuse 20 ummantelt werden.

Obwohl das in Fig. 2 gezeigte Gehäuse 20 einstückig ausgebildet dargestellt wird, kann das Gehäuse 20 auch mehrere Gehäuseteile umfassen, die nach einem Zusammenfügen der einzelnen Gehäuseteile zusammen mit den entsprechenden Abschnitten der Elektrodenleitungen 11 ummantelt werden. Die Ummantelung 25 des jeweiligen Gehäuses 20 erfolgt mit einem biokompatiblen Material, vorzugsweise mit einem Silikonkautschuk. Im Fall eines einteiligen Gehäuses 20, wie in Fig. 2 dargestellt, kann die Ummantelung 25 auch lediglich im oberen Bereich des Gehäuses 20 um die Elektrodenleitungen 11 herum erfolgen, solange eine fluiddichte Einheit zwischen dem Gehäuse 20 und den außerhalb des Gehäuses 20 liegenden Elektrodenleitungen 11 geschaffen wird.

Die Art der Ausgestaltung des medizinischen Implantats ist jedoch nicht auf die in der Figur gezeigte Ausführungsform beschränkt, sondern erstreckt sich auch auf Modifikationen und Variationen, im Bereich der angehängten Ansprüche.

## Patentansprüche

1. Medizinisches Implantat, insbesondere Herzschrittmacher, Kardioverter, Defibrillator oder dergleichen, mit:
zumindest einer funktionellen Einheit (2, 4), wie beispielsweise ein Energiespeicher (2), eine Steuerelektronik (4) oder Kombinationen daraus;
einem Gehäuse (20) zur Aufnahme der zumindest einen funktionellen Einheit (2, 4); und
zumindest einer Elektrode (10), die ein Anschlussende (12), eine Elektrodenleitung (11) und ein Stimulierungsende (13) aufweist,
**dadurch gekennzeichnet, dass** das Anschlussende (12) der Elektrode (10) in dem Gehäuse (20) zur Aufnahme der zumindest einen funktionellen Einheit (2, 4) angeordnet ist.

2. Medizinisches Implantat nach Anspruch 1, wobei das Gehäuse (20) einstückig ausgebildet ist.

3. Medizinisches Implantat nach Anspruch 1, wobei das Gehäuse (20) zumindest zwei Gehäuseteile umfasst.

4. Medizinisches Implantat nach Anspruch 3, wobei die Gehäuseteile an der Gehäuseinnenseite zumindest eine Struktur zur Montage der zumindest einen funktionellen Einheit (2, 4) aufweisen.

5. Medizinisches Implantat nach einem der Ansprüche 1 bis 4, wobei das Gehäuse (20) aus Kunststoff, vorzugsweise Polyurethan, ausgebildet ist.

6. Medizinisches Implantat nach einem der Ansprüche 1 bis 5, wobei das Gehäuse (20) zumindest teilweise mit einem elektrisch leitfähigen Material versehen ist.

7. Medizinisches Implantat nach einem der Ansprüche 1 bis 6, wobei das Anschlussende (12) der Elektrode (10) direkt mit der Steuerelektronik (4) verbunden ist.

8. Medizinisches Implantat nach Anspruch 7, wobei die Verbindung über Bonden ausgeführt ist oder eine Lötverbindung umfasst.

9. Medizinisches Implantat nach einem der Ansprüche 1 bis 8, wobei das Stimulierungsende (13) und zumindest ein Teil der Elektrodenleitung (11) außerhalb des Gehäuses (20) angeordnet sind, und das Gehäuse (20) und zumindest ein Abschnitt des außerhalb des Gehäuses (20) angeordneten Teils der Elektrodenleitung (11) eine Ummantelung (25) aufweisen.

10. Medizinisches Implantat nach Anspruch 9, wobei die Ummantelung (25) ein biokompatibles Material, vorzugsweise Silikonkautschuk, umfasst.

11. Medizinisches Implantat nach einem der Ansprüche 1 bis 10, wobei der Energiespeicher (2) einen Akkumulator und/oder einen Kondensator umfasst.

12. Medizinisches Implantat nach Anspruch 11, wobei der Energiespeicher (2) ausgestaltet ist, um mittels hochfrequenter Energie, magnetisch-induktiven Ladens, Wärme eines Patienten, chemischer Energie, mechanischer Bewegung oder Lungenbewegung wieder aufgeladen zu werden.

13. Medizinisches Implantat nach einem der Ansprüche 1 bis 12, wobei die Steuerelektronik (4) einen anwendungsspezifischen integrierten Schaltkreis umfasst.

14. Teilesatz, umfassend zumindest zwei medizinische Implantate nach einem der Ansprüche 1 bis 13.

15. Teilesatz nach Anspruch 14, wobei die zumindest zwei medizinischen Implantate unterschiedliche Ausgestaltungen von Elektroden (10), insbesondere des jeweiligen Stimulierungsendes (13), umfassen.

## Geänderte Patentansprüche

### Geänderte Patentansprüche gemäss Regel 137(2) EPÜ.

**1.** Kardio-Implantat, insbesondere Herzschrittmacher, Kardioverter, Defibrillator oder dergleichen, mit:
zumindest einer funktionellen Einheit (2, 4), wie beispielsweise ein Energiespeicher (2), eine Steuerelektronik (4) oder Kombinationen daraus;
einem Gehäuse (20) zur Aufnahme der zumindest einen funktionellen Einheit (2, 4); und
zumindest einer Elektrode (10), die ein Anschlussende (12), eine Elektrodenleitung (11) und ein Stimulierungsende (13) aufweist,
wobei das Anschlussende (12) der Elektrode (10) in dem Gehäuse (20) zur Aufnahme der zumindest einen funktionellen Einheit (2, 4) angeordnet ist, **dadurch gekennzeichnet, dass** das Anschlussende (12) direkt, vorzugsweise über Bonden oder eine Lötverbindung, an der funktionellen Einheit (2, 4) angebracht ist.

**2.** Kardio-Implantat nach Anspruch 1, wobei das Gehäuse (20) einstückig ausgebildet ist.

**3.** Kardio-Implantat nach Anspruch 1, wobei das Gehäuse (20) zumindest zwei Gehäuseteile umfasst.

**4.** Kardio-Implantat nach Anspruch 3, wobei die Gehäuseteile an der Gehäuseinnenseite zumindest eine Struktur zur Montage der zumindest einen funktionellen Einheit (2, 4) aufweisen.

**5.** Kardio-Implantat nach einem der Ansprüche 1 bis 4, wobei das Gehäuse (20) aus Kunststoff, vorzugsweise Polyurethan, ausgebildet ist.

**6.** Kardio-Implantat nach einem der Ansprüche 1 bis 5, wobei das Gehäuse (20) zumindest teilweise mit einem elektrisch leitfähigen Material versehen ist.

**7.** Kardio-Implantat nach einem der Ansprüche 1 bis 6, wobei das Anschlussende (12) der Elektrode (10) direkt mit der Steuerelektronik (4) verbunden ist.

**8.** Kardio-Implantat nach Anspruch 7, wobei die Verbindung über Bonden ausgeführt ist oder eine Lötverbindung umfasst.

**9.** Kardio-Implantat nach einem der Ansprüche 1 bis 8, wobei das Stimulierungsende (13) und zumindest ein Teil der Elektrodenleitung (11) außerhalb des Gehäuses (20) angeordnet sind, und das Gehäuse (20) und zumindest ein Abschnitt des außerhalb des Gehäuses (20) angeordneten Teils der Elektrodenleitung (11) eine Ummantelung (25) aufweisen.

**10.** Kardio-Implantat nach Anspruch 9, wobei die Ummantelung (25) ein biokompatibles Material, vorzugsweise Silikonkautschuk, umfasst.

**11.** Kardio-Implantat nach einem der Ansprüche 1 bis 10, wobei der Energiespeicher (2) einen Akkumulator und/oder einen Kondensator umfasst.

**12.** Kardio-Implantat nach Anspruch 11, wobei der Energiespeicher (2) ausgestaltet ist, um mittels hochfrequenter Energie, magnetisch-induktiven Ladens, Wärme eines Patienten, chemischer Energie, mechanischer Bewegung oder Lungenbewegung wieder aufgeladen zu werden.

**13.** Kardio-Implantat nach einem der Ansprüche 1 bis 12, wobei die Steuerelektronik (4) einen anwendungsspezifischen integrierten Schaltkreis umfasst.

**14.** Teilesatz, umfassend zumindest zwei Kardio-+-Implantate nach einem der Ansprüche 1 bis 13.

**15.** Teilesatz nach Anspruch 14, wobei die zumindest zwei Kardio-Implantate unterschiedliche Ausgestaltungen von Elektroden (10), insbesondere des jeweiligen Stimulierungsendes (13), umfassen.
